(19)

Europäisches
Patentamt
European
Patent Office
Office européen
des brevets

(11)     **EP 1 932 807 A1**

(12)                            **EUROPEAN PATENT APPLICATION**

(43) Date of publication:
     **18.06.2008   Bulletin 2008/25**

(51) Int Cl.:
     *C01G 49/00* $^{(2006.01)}$        *C07F 15/02* $^{(2006.01)}$
     *A61K 33/26* $^{(2006.01)}$        *B01J 20/06* $^{(2006.01)}$

(21) Application number: **06126122.8**

(22) Date of filing: **14.12.2006**

(84) Designated Contracting States:
     **AT BE BG CH CY CZ DE DK EE ES FI FR GB GR
     HU IE IS IT LI LT LU LV MC NL PL PT RO SE SI
     SK TR**
     Designated Extension States:
     **AL BA HR MK RS**

(71) Applicant: **Novartis AG
     4056 Basel (CH)**

(72) Inventor: **The designation of the inventor has not
     yet been filed**

(74) Representative: **von Sprecher, Georg et al
     Novartis AG
     Corporate Intellectual Property
     4002 Basel (CH)**

(54)     **Inorganic compounds**

(57)     The present invention relates to a new iron containing phosphate absorber and its use e.g. for treating hyper-phosphataemia.

EP 1 932 807 A1

**Description**

The present invention relates to new iron containing phosphate absorbent, process for its production, uses thereof and pharmaceutical compositions containing it.

Phosphorus is critical for bone mineralization, cellular structure, genetic coding, and energy metabolism. Many organic and inorganic forms exist. Phosphorus is present in nearly all foods, and GI absorption of dietary forms is very efficient. Phosphorus homeostasis normally is maintained through several mechanisms (renal excretion, cellular release, hormonal control, etc). When the phosphorus load (from GI absorption, exogenous administration, or cellular release) exceeds renal excretion and tissue uptake, hyperphosphatemia occurs.

Hyperphosphatemia is associated with significant increase in morbidity and mortality, and may induce severe complications, such as hypocalcemia, decreasing of vitamin D production, metastatic calcification. Hyperphosphatemia is also contributing to the increased incidence of cardiovascular disease among dialysis-dependent patients, and can result in bone pathology.

At least 70% of patients with renal insufficiency or renal failure show hyperphosphatemia. In many cases, restricting intake of dietary phosphorus is not sufficient to reduce serum phosphate levels into the normal range, and oral phosphate binders need to be taken.

Calcium and aluminium salts orally taken as treatment for hyperphosphatemia are known. But there are concerns regarding their long-term safety. The traditional aluminium-based phosphate binders have the drawback of side effects due to aluminium absorption (osteomalacia, encephalopathy, microcytic anaemia). Calcium-containing phosphate binders (calcium carbonate or calcium acetate) may aggravate metastatic calcification, particularly if they are taken together with vitamin D analogues and a high calcium dialysate concentration.

An iron-based ferric citrate phosphate binder, known as Zerenex™, is described in US 6,903,235B. Zerenex™ is an oral, inorganic, iron-based compound that has the capacity to bind phosphorous and form non-absorbable complexes. Since this product is soluble its long term administration may induce an increase of the concentration of iron in gastrointestinal tract, which may be safety issue as mentioned hereinabove.

Sevelamer, a synthetic polymer commercialized by Genzyme under the name of Renagel®, 2-propen 1-amine, hydrochloride, polymer with (chloromethyl)oxirane 9Cl) is an iron exchange gel matrix.

Iron containing compounds for the absorption of phosphate are known in the art. For example, US 6,174,442, the content thereof being enclosed herewith by reference, describes an absorbent for phosphate, which contains $\beta$-iron hydroxide stabilized by carbohydrates and/or humic acid.

The possible release of iron under physiological conditions from any iron containing drugs or compounds, in particular in case of the phosphate absorber described in US 6,174,442, may be a safety issue, since excess iron is toxic to body organs. Preferably the daily release of iron should not be higher than 20mg iron per day.

A too high release of iron is particularly a safety issue in case of patients suffering from haemochromatosis. Haemochromatosis is a very common genetic disorder of iron metabolism wherein absorption of iron through the intestine is uncontrolled even when body saturation levels have been reached.

The phosphate binders available in the prior art bind not more than about 120mg phosphate per gram of product. Because of the relatively low absorption capacity of the phosphate absorbers available in the prior art, the amount of absorber, e.g. the amount and/or number of oral dosage forms containing it to be taken every day is high. For example the average daily dosage to be taken by dialysis patients in order to avoid/treat hyperphosphatemia is of about 9 capsules in case of Zerenex™, and of 14 capsules in case of Renagel®. This indicates that the patient compliance of the phosphate absorbers available in the prior art is very low.

Therefore there is a need to provide phosphate binders with higher phosphate binding capacity in order to reduce the amount of product to be ingested, e.g. to improve the patient compliance.

Furthermore there is a need to provide phosphate absorber which does not release iron, or does release only small amounts of iron under physiological conditions, e.g. an iron amount smaller than the daily acceptable dosage of 20mg iron.

There is a need to provide phosphate absorber which is has a low bioavailability, preferably which is not bioavalaible.

The present invention provides a new phosphate absorber containing iron (III) oxide-hydroxide, preferably $\alpha$-, $\gamma$- and/or $\delta$- iron (III) oxide-hydroxide, even more preferably $\gamma$-iron (III) oxide-hydroxide.

The preparation of iron oxide-hydroxides is known in the prior art and is described in U. Schwertmann and M. R. Cornell "Iron oxides in the Laboratory", Wiley-VCH, Second, completed revised and extended edition, 1991. This document teaches that it is possible to prepare $\beta$-iron (III) hydroxide. i.e. to obtain iron (III) hydroxide with the $\beta$-configuration from iron (III), and to prepare $\gamma$-iron (II) oxide-hydroxide, i.e. to obtain iron oxide-hydroxide having $\gamma$-configuration from iron (II).

Surprisingly, it has been found that it is possible to obtain $\gamma$-iron (III) oxide-hydroxide, i.e. to obtain from iron (III) iron oxide-hydroxide with the $\gamma$-configuration.

It is known that small changes in the reaction conditions used to prepare iron oxide-hydroxide, such as pH, OH/Fe (ratio base to iron), iron concentration, etc, may prevent the desired product from being obtained (see page 65 of U. Schwertmann and M. R. Cornell). In particular simply scaling the quantities up or down to produce different amounts, e.g. higher amounts, of iron oxide-hydroxide may result in the production of a product having a different structure..

There is a need to provide phosphate absorbers with are stable products usable as pharmaceuticals, e.g. without microbial contaminations and/or which are suitable for oral administration, i.e. can be formulated into oral pharmaceutical compositions, e.g. which are not dusty, free- flowing and thus can be filled e.g. into sachets or stick packs, e.g. can be mecanically filled.

In addition, there is a need to provide a product which can be manufactured in a large scale, in particular which can be mechanically filled into sachets or stick packs.

Surprisingly, it has been found that it is possible to obtain absorbent for phosphate containing iron(III) oxide-hydroxide which has improved properties in comparison to prior art compounds, e.g. to the absorbent for phosphate described in US 6,174,442, e.g. which shows significant higher phosphate binding capacity.

According to the invention there is provided a new iron (III) oxide-hydroxide containing compound, further comprising starch and sucrose, for the absorption of phosphate, which has higher phosphate binding capacity than the phosphate absorbents of the prior art, as herein defined as "compound of the invention".

Higher phosphate binding capacity means about 15% higher than the phosphate absorbers known in the art, e.g. 20% higher, e.g. 25% e.g. than the absorber described in US 6,174,442 For example, the compound of the invention may absorb more than 12%m/m phosphate, preferably more than 14%m/m,.

Preferably the compound of the invention comprises γ-iron(III) oxide-hydroxide.

Preferably, the compound of the invention is x-ray amorphous

Preferably the iron oxide-hydroxide contained in the compound of the invention is a complex, preferably an insoluble complex, e.g. under physiological conditions. The complex may be stabilized by sucrose.

In another embodiment of the invention, the compound of the invention comprises starch particles covered by iron (III) oxide-hydroxide and optionally stabilized by sucrose. Preferably the iron may be homogenously distributed on the surface of the particles.

In yet a further aspect of the invention there is provided a new phosphate absorber containing iron (III) oxide-hydroxide, starch and sucrose which releases little iron in simulated gastric fluids (as defined in pharmacopeia). For example, the compound of the invention does not significantly release iron at a pH range of 2 to 10, In one example, 10 g of the compound of the invention may release less than 20mg of iron at pH above 2.

In yet a further aspect of the invention the compound of he invention may contain about 10 to about 35wt%, e.g. about 20 to about 30 wt%, preferably at least about 20 weight % of starch, for example about 28 weight % of starch, based on the total weight of the compound.

In yet a further aspect of the invention there is provided a new phosphate absorber containing iron (III) oxide-hydroxide and starch, which contains at least about 14 weight % of sucrose, for example about 28wt%, based on the total weight of the compound.

In yet a further aspect of the invention there is provided a new phosphate absorber containing iron(III) oxide-hydroxide, which contains more than about 5 weight% of water, for example about 5 to about 10 weight%, for example about 5 to about 8 weight %, based on the total weight of the compound.

In yet a further aspect of the invention there is provided a new phosphate absorber containing iron (III) oxide-hydroxide, which contains at least about 20 weight% of iron, for example at least about 25 weight %, for example about 30 weight% of iron, based on the total weight of the product. In another aspect of the invention the iron content of the compound of the invention is about 20 to about 30 weight %, for example about 25 to about 30 weight %, based on the total weight of the product.

In yet another aspect of the invention there is provided a new phosphate absorber containing γ-iron (III)oxide-hydroxide and further comprising starch and sucrose, which contains about 5 to about 10 weight% of water, about 20 to about 30 weight% of iron, based on the total weight of the compound.

The present invention also includes a process for the preparation of y-iron(III) oxide-hydroxide, which process comprises reacting an aqueous solution of iron(III)chloride with an aqueous basic mixture of a base and starch, at ambient temperature.

The present invention also includes a process for the preparation of a phosphate absorber comprising iron(III) oxide-hydroxide, which process comprises the steps of

i) simultaneously mixing an aqueous solution of iron(III)chloride with a aqueous base at pH comprised between 6 and 8, preferably around 7,

ii) adding starch while maintaining the solution at a constant pH between 6 and 8, and optionally

iii) isolating the solids and washing with water,

iv) adding sucrose and an alcohol, e.g. ethanol, and optionally other binders for drug product, and optionally

v) isolating the product by spray drying or fluidized spray drying.

In yet a further aspect of the invention the manufacturing process of the phosphate absorber of the invention may use solid iron(III)chloride hexahydrate.

The base to be used can be LiOH, KOH, NaOH, Ca(OH)$_2$, Mg(OH)$_2$, Li$_2$CO$_3$, K$_2$CO$_3$, CaCO$_3$, MgCO$_3$, preferable Na$_2$CO$_3$.

According to the invention, step ii) is preferably made at a temperature between about 1 and 20, preferably 2 to 10, preferably about 5°C.

In step iv), the alcohol to be used is e.g. ethanol, methanol, 2-propanol.

In step iv), the sucrose may be replaced by or be mixed to another carbohydrate, for example maltodextrin.

Subsequently to step iv) the suspension may stagnate, with or without stirring, e.g. more than 1 hour, preferably during 1 to 5 hours, more preferably over night.

Isolation of the product s in step iii) may be made by filtration or decandation.

According to the invention, in step iii) the iron (III) oxide-hydroxide containing compound is washed, e.g. with water or an aqueous solution of NaHCO$_3$, preferably with water. Combinations of water washings and NaHCO$_3$-washings may be used.

According to the invention, washing may be done e.g. by decantation, filtration, centrifugation, preferably by decantation.

Washing can be done until the level of impurities is down to a predefined level, e.g. from a few hours up to a few days. Preferably 2 to 5 washings are done, more preferably 3 to 5.

The product is then resuspended in water. A minimum amount of water is needed so that the suspension can be processable. For example the ratio amount of water/final phosphate absorber may be from about 0.8 to about 2, preferably 1.1. to 1.5, more preferably about 1.

According to the invention, the amount of sucrose added in step iv) may be of about 5 to about 15%wt, preferably about 5 to about 10, based on the weight of the phosphate absorber.

In step i), a solution of iron (III)chloride at about 20 to 30 wt/wt%, preferably about 25 wt/wt%, may be used.

In step i), a base solution of about 20 to 30, e.g. about 22 to 27, e.g. about 25.5vol% may be used.

Starch may be e.g. a mixture of different starch , e.g. a mixture of potato starch and soluble starch or maltodextrin, e.g. a mixture of 80 wt% or more of potato starch and 20wt % or less of soluble starch or maltodextrin.

Soluble carbohydrates, e.g. sugars, can be selected from agarose, dextran, dextrin, dextran derivatives, cellulose, cellulose derivatives, sucrose, maltose, lactose, mannitol and mixture thereof, preferably carbohydrates are sucrose, maltodextrin or mixture thereof.

The present invention provides a free-flowing, dust-free, coarse material which can be filled into sachets or stick packs, e.g. automatically filed. A subsequent tableting step may be performed. A tablet form may be produced by dry compression, e.g. direct compression or roller compaction.

According to the present invention, pharmaceutical composition of the invention refers to pharmaceutical composition containing the compound of the invention.

The pharmaceutical composition of the invention may contain a dry binder, e.g. a microcrystalline cellulose (MCC); a glidant, e.g. Mg- stearate or hydrophilic glidant , such as PEG 6000 or PEG 4000 in concentrations of up to 5%.

## Examples

The following examples are illustrative of the invention.

## Example 1:

An aqueous solution of 21.1g sodium carbonate dissolved in 105g water (solution A) and an aqueous solution of 26.5g iron(III) chloride hexahydrate dissolved in 55g water (solution B) are prepared. Solution A (4.2g/min) and B (2.7g/min) are dosed to a suspension of 7.36g potato starch in 40g water over a time span of 30 minutes by continuous mixing of solution A and B and subsequent addition to the potato starch suspension. The resulting brown-reddish suspension is stirred for at least one hour at 25°C, filtered and washed three times with 173.6g water each. To the resulting reddish brown solids 14.6g water, 7.36g sucrose and 24.2g ethanol are added and the mixture is stirred for 60 minutes. 18.4g of the final product are obtained by fluidized spray drying using appropriate FSD conditions.

**Example 2:** phosphate binding capacity of the material described in Example 1 determined by ion chromatography/ conductivity detection.

Separation mechanism used is ion exchange.
Reagents: Highly pure water (e.g. from Milli-Q-System (Millipore)); Sodium hydroxide (50% (m/m) solution, carbonate free, e.g. Merck 1.58793); 0.1 N Hydrochloric acid (HCl); Hydrochloric acid 37%; Sodium hydrogen phosphate (Na2HP04).
Equipment: Ion chromatograph with gradient pump; Anion exchange column (e.g. Dionex IonPac AS11-HC Length 250 mm, internal diameter 4 mm; or equivalent); Self regenerating anion suppressor (e.g. Dionex ASRS-ULTRA II 4 mm, anion suppressor system), PVDF Filter (e.g. Infochroma: 8817E-PV-4 ECO HPLC-Filter PVDF 0.45 $\mu$m). Chromatographic conditions
Gradient elution using degassed water as eluent 1 and 80 mM sodium hydroxide in water as eluent 2.

Gradient program

| | Time | OH⁻ [mM] | % E1 | % E2 | Remark |
|---|---|---|---|---|---|
| | 0 10 | 16 80 | 80 0 | 20 100 | Run start |
| | 14 | 80 | 0 | 100 | |
| | 14.1 | 16 | 80 | 20 | |
| | 16.0 | 16 | 80 | 20 | Run end |
| | 18.0 | 16 | 80 | 20 | |
| Flow rate | 0.6 mL/min | | | | |
| Detection | Suppressed electrical conductivity | | | | |
| Column temperature | 30°C | | | | |
| Injection volume | 25 $\mu$L | | | | |

System suitability test (SST)

The system suitability test proves that chloride and phosphate are sufficiently separated. Moreover the repeatability of injection is shown by repetitive injection of the comparison solution for calculation. A blank chromatogram shows that no interfering peak exist in the solvent. A 1/100 dilution of the comparison solution for calculation is used to show sensitivity of the system.

Preparation of solutions

| | |
|---|---|
| Test solution (prepare in duplicate TSx.1 and TSx.2) | Accurately weigh (to within 0.01 mg) 365.0 to 385.0 mg of the test substance into a 25 mL volumetric flask. Add 20.0 mL of CS1 to this substance and let stand for 10 min. Adjust the pH of this suspension to pH 2.0 with HCl 0.1 N and fill to the mark with water. The tightly closed flask is then kept in a water bath of 37°C for 2 hours. Approximately, every half an hour the suspension is stirred manually by shaking the vial. |

(continued)

| Comparison stock solution (prepare single: CS1) | Afterwards, filter approximately 2.0 mL of the resulting suspension through a 0.45 $\mu$m filter (PVDF) into a vial. Take 1.0 mL of this filtrate into a 25 mL volumetric flask and fill to the mark with water. The clear and colorless solution will be used directly for chromatography. Concentration ($PO_4^{3-}$) level for CS1 = 4273 $\mu$g/mL Preparation: Accurately weigh (to within 0.1 mg) 6350 mg to 6425 mg of $Na_2HPO_4$ into a 1000 mL volumetric flask, dissolve with app. 900 mL water and set to pH 2.0 with HCl 37% and 0.1 N. Then fill to the mark with solvent. |
| Comparison solution for calculation (prepare single: CS2) | Concentration ($PO_4^{3-}$) level for CS2 = 170.9 $\mu$g/mL Preparation: Pipette 2.0 mL of CS1 into a 50 mL volumetric flask and fill to the mark with solvent. |

Evaluation/assessment

The analyte peaks in the chromatograms of the test solution is identified by comparing the retention times with those of the peaks in the chromatograms of the comparison solutions.
The peak areas for each analyte peak is determined in the chromatograms of the test solutions. The peak areas for each analyte peak is also determined in the chromatograms of the CS2 solutions.

Calculation

**Per cent absorption (%) (m/m) of phosphate**

1.

$$ABS_{PO_4}(\%; \mathrm{m/m}) = \frac{m_{PO_4 added} - m_{PO_4 measured}}{EW_{TS}} \times 100\%$$

2.

$$m_{PO_4 added}[mg] = EW_{Na_2HPO_4} \times \frac{MW_{PO_4^{3-}}}{MW_{Na_2HPO_4}} \times \frac{1}{50}$$

3.

$$m_{PO_4 measured}[mg] = EW_{Na_2HPO_4} \times \frac{MW_{PO_4^{3-}}}{MW_{Na_2HPO_4}} \times \frac{1}{40} \times \frac{PA_{TS}}{PA_{CS2}}$$

$EW_{TS}$ — Weight of the **material described in Example 1** in test solution [mg]
$EW_{Na2HPO4}$ — Weight of sodium hydrogen phosphate in CS1 solution [mg]
PATS — Peak area of phosphate in test solution
$PA_{CS2}$ — Peak area of phosphate in comparison solution 2
$MW_{PO4}^{3-}$ — Molecular weight of phosphate: 94.97 g/mol
$MW_{Na2HPO4}$ — Molecular weight of sodium hydrogen phosphate: 141.96 g/mol

Results:

The compound absorbs 20% m/m phosphate.

The phosphate absorbent according to the invention exhibit valuable pharmacological properties, e.g. absorbing inorganic phosphate or phosphate bound to foodstuffs from body fluids or foodstuffs, e.g. as indicated in *in vitro* and *in vivo* tests and are therefore indicated for therapy.

The phosphate absorbents according to the invention are, therefore, useful in the treatment and/or prevention of hyperphosphataemia, hypercalcaemia, hyperparathyroidism reduction, in cardiovascular morbidity and mortality, renal osteodystrophy, calciphylaxis and soft tissue calcifications.

The compounds of the invention are more particularly useful in patients with hyperphosphataemia, e.g. for dialysis-dependent patients, e.g. hemodialysis, or patients suffering from advanced chronic kidney diseases (CKD), chronic renal failure, chronic renal insufficiency, end-stage renal disease.

The phosphate absorbent according to the invention may be administered by any conventional route, in particular enterally, e.g. orally, e.g. in the form of tablets or capsules.

Pharmaceutical compositions comprising the compound of the invention in association with at least one pharmaceutical acceptable carrier or diluent may be manufactured in conventional manner by mixing with a pharmaceutically acceptable carrier or diluent.

Unit dosage forms for oral administration contain, for example, from about 0.5 to 2, preferably 1 to 1.5, more preferably about 1.25 g of active substance.

The phosphate absorbent according to the invention may also be used for the absorption of phosphate bound to foodstuffs. They may be admixted with foodstuffs.

In accordance with the foregoing the present invention provides:

1.1 A phosphate absorbent containing iron(III) oxide-hydroxide, starch and sucrose, which contains from about 25 to 35 weight% of iron, about 5 to 10 weight % of water, based on the total weight of the absorbent, the iron(III) oxide-hydroxide being preferably .γ-iron(III).

2.1 A method for preventing or treating disorders or diseases such as indicated above, in a subject in need of such treatment, which method comprises administering to said subject an effective amount of a phosphate absorbent according to the invention;

2.2 A method for controlling serum phosphate and serum calcium-phosphate product levels, while maintaining normal serum calcium levels, in a subject in need of such treatment, e.g. in patients on chronic hemodialysis, which method comprises administering to said subject an effective amount of a phosphate absorbent according to the invention;

2.3 A method for selectively removing inorganic phosphate or eliminating inorganic phosphate, e.g. from dialysis fluids, whole blood, plasma, in a subject in need of such treatment, e.g. in patients on chronic hemodialysis, which method comprises administering to said subject an effective amount of a phosphate absorbent according to the invention.

2.4 A method for selectively removing inorganic phosphate bound to foodstuffs.

3.1 A phosphate absorbent according to the invention for use as a pharmaceutical, e.g. in any of the methods as indicated under 2.1 to 23 above.

4.1 A pharmaceutical composition, e.g. for use in any of the methods as in 2.1 to 2.3 above comprising a phosphate absorbent according to the invention in association with a pharmaceutically acceptable diluent or carrier therefor.

4.2 A pharmaceutical composition, e.g. for use as a pharmaceutical preparation for the selective elimination of inorganic phosphate from liquids, wherein the composition contains an phosphate absorbent material according to the invention

5. A phosphate absorbent according to the invention for use in the preparation of a pharmaceutical composition for use in any of the method as in 2.1 to 2.3 above.

6. A pharmaceutical composition suitable for oral administration containing the phosphate absorbent according to the invention.

The compound of the invention may be administered as the sole active ingredient or together with another Phosphate reducing agent, such as sevelamer; Fosrenol; Ca acetate; or Ca carbonate. It may also be administered in combination with a calcimimetic such as cinacalcet; vitamin D; or calcitriol.

In accordance with the foregoing the present invention provides in a yet further aspect:

7. A method as defined above comprising co-administration, e.g. concomitantly or in sequence, of a therapeutically effective amount of a phosphate absorbent according to the invention, and a second drug substance, said second drug substance being another Phosphate reducing agent, a calcimimetic, vitamin D, or calcitriol, e.g. as indicated above.

8. A therapeutic combination, e.g. a kit, comprising a) a phosphate absorber according to the invention, and b) at least one second agent selected from an another Phosphate reducing agent, a calcimimetic, vitamin D and calcitriol. Component a) and component b) may be used concomitantly or in sequence. The kit may comprise instructions for its administration.

Where a phosphate absorber according to the invention is administered in conjunction with another Phosphate reducing agent, such as sevelamer, Fosrenol, Ca acetate or Ca carbonate; a calcimimetic such as cinacalcet; or with vitamin D or calcitriol, e.g. for preventing or treating hyperphosphataemia or other diseases or disorders as hereinabove specified, dosages of the co-administered compound will of course vary depending on the type of co-drug employed, on the condition being treated and so forth.

**Claims**

1. An iron containing compound for the absorption of phosphate as herein defined and/or described, a process for its preparation, its use as a pharmaceutical, a pharmaceutical composition containing such a compound, a method of treatment or prevention using such a compound, or a pharmaceutical combination comprising such a compound, substantially as herein defined and/or described.

2. A compound substantially as described in the Examples.

3. A solid dosage form containing the compound of the invention.

4. A process for the preparation of y-iron(III) oxide-hydroxide as herein defined and/or described,

5. A process for the preparation of a phosphate absorber according to the invention as herein defined and/or described.

**European Patent Office**

**EUROPEAN SEARCH REPORT**

Application Number

EP 06 12 6122

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 92/01458 A (PURDUE RESEARCH FOUNDATION [US]) 6 February 1992 (1992-02-06) * the whole document * * claim 1 * ----- | 1-5 | INV. C01G49/00 C07F15/02 A61K33/26 B01J20/06 |
| X | EP 0 600 347 A2 (BRAUN MELSUNGEN AG [DE] BRAUN MELSUNGEN AG [US] SEBO GMBH [DE]) 8 June 1994 (1994-06-08) * examples 1-12 * * the whole document * ----- | 1-5 | |
| X | WO 97/22266 A (VIFOR INT AG [CH]; GEISSER PETER [CH]; PHILIPP ERIK [CH]) 26 June 1997 (1997-06-26) * examples 1-16 * * the whole document * ----- | 1-5 | |
| X | CA 2 240 668 C (VIFOR INT AG [CH]) 26 June 1997 (1997-06-26) * claims 1-16 * ----- | 1-5 | |
| X | DE 10 2004 031181 A1 (VIFOR INTERNATIONAL AG ST GALL [CH]) 19 January 2006 (2006-01-19) * paragraphs [0007] - [0020] * * the whole document * ----- | 1-5 | TECHNICAL FIELDS SEARCHED (IPC) C01G C07F A61K B01J |
| X | WO 2006/000547 A (VIFOR INT AG [CH]; MUELLER HANS-MARTIN [CH]; PHILIPP ERIK [CH]; GEISSE) 5 January 2006 (2006-01-05) * examples 1-5 * * the whole document * ----- | 1-5 | |
| X | US 5 514 281 A (BOOS KARL-SIEGFRIED [DE] ET AL) 7 May 1996 (1996-05-07) * examples 1-12 * * the whole document * ----- | 1-5 | |

-/--

The present search report has been drawn up for all claims

| Place of search | Date of completion of the search | Examiner |
|---|---|---|
| Munich | 15 May 2007 | Bader, Karl Günther |

EPO FORM 1503 03.82 (P04C01)

European Patent
Office

**EUROPEAN SEARCH REPORT**

Application Number

EP 06 12 6122

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/15189 A (CROSFIELD JOSEPH & SONS [GB]; ROBERTS NORMAN BRYSON [GB]; WEBB MAURICE) 1 April 1999 (1999-04-01) * the whole document * ----- | 1-5 | |
| X | EP 1 457 256 A (MUROMACHI CHEMICAL CO LTD [JP]) 15 September 2004 (2004-09-15) * the whole document * ----- | 1-5 | |
| | | | TECHNICAL FIELDS SEARCHED (IPC) |

The present search report has been drawn up for all claims

| Place of search | Date of completion of the search | Examiner |
|---|---|---|
| Munich | 15 May 2007 | Bader, Karl Günther |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

    .................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 06 12 6122

This annex lists the patent family members relating to the patent documents cited in the above-mentioned European search report.
The members are as contained in the European Patent Office EDP file on
The European Patent Office is in no way liable for these particulars which are merely given for the purpose of information.

15-05-2007

| Patent document cited in search report | | Publication date | Patent family member(s) | | Publication date |
|---|---|---|---|---|---|
| WO 9201458 | A | 06-02-1992 | US | 4970079 A | 13-11-1990 |
| EP 0600347 | A2 | 08-06-1994 | AT | 199322 T | 15-03-2001 |
| | | | DE | 4239442 A1 | 01-06-1994 |
| | | | ES | 2155064 T3 | 01-05-2001 |
| | | | JP | 3406360 B2 | 12-05-2003 |
| | | | JP | 7075669 A | 20-03-1995 |
| WO 9722266 | A | 26-06-1997 | AT | 207707 T | 15-11-2001 |
| | | | AU | 1374797 A | 14-07-1997 |
| | | | BR | 9612138 A | 13-07-1999 |
| | | | DE | 19547356 A1 | 26-06-1997 |
| | | | DK | 868125 T3 | 19-11-2001 |
| | | | EP | 0868125 A1 | 07-10-1998 |
| | | | ES | 2171758 T3 | 16-09-2002 |
| | | | IN | 190206 A1 | 28-06-2003 |
| | | | JP | 2000506372 T | 30-05-2000 |
| | | | JP | 3665345 B2 | 29-06-2005 |
| | | | NO | 982722 A | 18-08-1998 |
| | | | PT | 868125 T | 29-04-2002 |
| | | | TR | 9801152 T2 | 21-09-1998 |
| | | | TW | 457090 B | 01-10-2001 |
| | | | US | 6174442 B1 | 16-01-2001 |
| | | | ZA | 9610643 A | 24-06-1997 |
| CA 2240668 | C | 04-07-2006 | CA | 2240668 A1 | 26-06-1997 |
| DE 102004031181 | A1 | 19-01-2006 | AR | 049522 A1 | 09-08-2006 |
| | | | AU | 2005256617 A1 | 05-01-2006 |
| | | | CA | 2571364 A1 | 05-01-2006 |
| | | | WO | 2006000547 A2 | 05-01-2006 |
| WO 2006000547 | A | 05-01-2006 | AR | 049522 A1 | 09-08-2006 |
| | | | AU | 2005256617 A1 | 05-01-2006 |
| | | | CA | 2571364 A1 | 05-01-2006 |
| | | | DE | 102004031181 A1 | 19-01-2006 |
| US 5514281 | A | 07-05-1996 | NONE | | |
| WO 9915189 | A | 01-04-1999 | AT | 332699 T | 15-08-2006 |
| | | | AU | 9173398 A | 12-04-1999 |
| | | | BR | 9812223 A | 18-07-2000 |
| | | | CA | 2303820 A1 | 01-04-1999 |
| | | | CN | 1270526 A | 18-10-2000 |
| | | | CN | 1911245 A | 14-02-2007 |
| | | | DK | 1015002 T3 | 13-11-2006 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**

EP 06 12 6122

This annex lists the patent family members relating to the patent documents cited in the above-mentioned European search report.
The members are as contained in the European Patent Office EDP file on
The European Patent Office is in no way liable for these particulars which are merely given for the purpose of information.

15-05-2007

| Patent document cited in search report | | Publication date | Patent family member(s) | | Publication date |
|---|---|---|---|---|---|
| WO 9915189 | A | | EP | 1759704 A2 | 07-03-2007 |
| | | | EP | 1015002 A1 | 05-07-2000 |
| | | | HU | 0004527 A2 | 28-04-2001 |
| | | | JP | 2001517633 T | 09-10-2001 |
| | | | NZ | 503136 A | 27-04-2001 |
| | | | PL | 339079 A1 | 04-12-2000 |
| | | | PT | 1015002 T | 31-10-2006 |
| | | | US | 6926912 B1 | 09-08-2005 |
| EP 1457256 | A | 15-09-2004 | AU | 2002357602 A1 | 09-07-2003 |
| | | | CN | 1606472 A | 13-04-2005 |
| | | | WO | 03053565 A1 | 03-07-2003 |
| | | | US | 2005107253 A1 | 19-05-2005 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6903235 B

- US 6174442 B

**Non-patent literature cited in the description**

- **U. SCHWERTMANN ; M. R. CORNELL.** Iron oxides in the Laboratory. Wiley-VCH, 1991